# EUROPÄISCHE PATENTANMELDUNG

(11) **EP 0 848 949 A1**
(43) Veröffentlichungstag der Anmeldung: **24.06.1998**
(21) Anmeldenummer: 97122419.1
(22) Anmeldetag: 18.12.1997
(51) Int. Cl.: A61K 9/12, A61K 31/07

(54) **Aerosol enthaltend Vitamin A oder ein Derivat davon**

(30) Priorität: 18.12.1996 DE 19652790
(71) Anmelder: HERMES Fabrik pharmazeutischer Präparate Franz Gradinger GmbH & Co., 82049 Grosshesselohe/München (DE)
(72) Erfinder: Thoma, Karl Prof. Dr., 82152 Krailling (DE); Rothenberger, Siegfried Dr., 81475 München (DE); Hein, Thomas Dr., 83623 Dietramszell (DE)
(74) Vertreter: VOSSIUS & PARTNER

(57) **Zusammenfassung**

Die Erfindung betrifft pharmazeutische Zusammensetzungen in Form eines Aerosols zur Anwendung auf Schleimhäuten der Atemwege, enthaltend:
a) einen Wirkstoff oder eine Wirkstoffkombination aus der Gruppe der Retinoide und Carotinoide,
b) ein verflüssigtes Treibgas oder Treibgasgemisch aus der Gruppe der partiell bis vollständig fluorierten, jedoch chlorfreien Kohlenwasserstoffe,
c) ein verflüssigtes Treibgas oder Treibgasgemisch als Lösungsvermittler mit Aerosolisierungseffekt aus der Gruppe der gesättigten Kohlenwasserstoffe,
d) gegebenenfalls antioxidativ wirkende und/oder weitere übliche pharmazeutische Hilfsstoffe, welche sich für Aerosolformulierungen eignen.

## Beschreibung

Der Begriff Vitamin A steht für eine Reihe chemisch ähnlicher Verbindungen mit unterschiedlicher Wirkung im menschlichen und tierischen Organismus. Das Vitamin ist für den Menschen essentiell, d.h. es entsteht ein Vitaminmangel, wenn es nicht mit der Nahrung zugeführt wird. Der Vitamin-A-Mangel äußert sich in verschiedenen Veränderungen im Bereich von Haut, Schleimhäuten und Augen. Symptome können beispielsweise Verhornung der Schleimhäute der Atemwege oder der Bindehaut des Auges, erhöhte Infektanfälligkeit sowie bei ausgeprägtem Mangel die Erblindung sein. Bei Zufuhr des Vitamins sind die meisten mangelbedingten Veränderungen vor allem im Bereich der Schleimhäute rückbildungsfähig. Jedoch müssen bei systemischer Anwendung zur Erreichung einer Rückbildung, beispielsweise einer Plattenepithelmetaplasie, oder zur Verhinderung eines erneuten Auftretens solcher Veränderungen hohe Konzentrationen eingesetzt werden, die teilweise zu erheblichen Nebenwirkungen (Hirndrucksymptomatik, Leberzellstoffwechselstörungen, u.a.) führen. Außerdem verbietet sich der Einsatz hochdosierter Präparate in der Schwangerschaft aufgrund des Risikos fetaler Mißbildungen (Bauernfeind J. C.; The Safe Use Of Vitamin A, The Nutrition Foundation, Washington D.C. 1980).

Des weiteren ist eine Zufuhr von Vitamin A auch in physiologischer Konzentration bei Störungen des Leberzellstoffwechsels, wie z.B. Entzündung oder Zirrhose, nicht zulässig, da durch die gleichzeitig gestörte Proteinsynthese (mangelnde Bildung des Transportproteins) der Leber eine Ausschleusung des Vitamins aus Speichern, in die es nach Resorption überführt wird, nicht möglich ist. Hinzu kommt, daß durch die peripheren Zielgewebe, wie z.B. das Respirationsepithel das Vitamin nur dann nach systemischer Gabe aufgenommen und seiner Funktion zugeführt werden kann, wenn es an eben dieses Transportprotein gebunden ist.

In der EP-A-0 352 412 wird zur Lösung dieser Problematik die Verwendung einer Zubereitung von Estern des Retinols sowie der Retinsäure zur inhalativen Applikation beschrieben. Hierdurch wird insbesondere eine topische Einwirkung des Wirkstoffs auf die Schleimhäute des Tracheo-Bronchialtraktes von Mensch und Tier ermöglicht. Damit wird die Vorbeugung und Behandlung von bestimmten Erkrankungen und Funktionsanomalien erleichtert, beispielsweise bestimmter zellulärer Differenzierungsstörungen, Plattenepithel-Metaplasien, neoplastischen Veränderungen, eingeschränkter Aktivität des Flimmerepithels und Dysfunktion schleimbildender Zellen. Weiterhin kann diese Zubereitung für die Therapie oder als Adjuvans bei der Therapie v.u.a. Bronchialkarzinomen, akuten und chronischen Bronchitiden und der bronchiopulmonalen Dysplasie von Neugeborenen eingesetzt werden.

Neben den in der EP-A-0 352 412 genannten Retinylestern sind jedoch auch eine Reihe ähnlicher Stoffe aus der Substanzklasse der Retionide ebenso in der Lage, die beschriebenen Wirkungen auszuüben. Weiterhin sind Stoffe aus der Substanzklasse der Carotinoide mit Provitamin-A-Wirkung, wie z.B. Betacaroten, dazu befähigt, der Vitamin-A-Versorgung des Menschen zu dienen. Im Körper werden diese Stoffe zur eigentlichen Wirkform umgewandelt. Alle die genannten Stoffe zeichnen sich durch ähnliche chemische Eigenschaften, beispielsweise lipophiles Lösungsverhalten, aus.

EP-A-0 352 412 offenbart u.a. Beispiele pharmazeutischer Zubereitungen, bei denen der oder die Wirkstoffe im unter Druck verflüssigten Treibgas einer Aerosol-Treibgaspackung vorliegen. Dies ist eine bevorzugte Zubereitung, da die handelsüblichen und vor allem im medizinischen Bereich meist verwendeten FCKW-Treibmittel gleichzeitig als sehr gutes Lösungsmittel für die lipophilen Wirkstoffe dienen. Nachdem jedoch gegen Ende der siebziger Jahre erkannt wurde, daß diese Treibgase mit für die Schädigung der Ozonschicht verantwortlich sind, bemühte man sich zunehmend, ihre Anwendungen zu reduzieren. Seit dem sogenannten Montrealer Abkommen wird die technische Verwendung der FCKW-Treibgase in vielen Ländern vom Gesetzgeber reglementiert. In Deutschland ist seit 1991 die sogenannte FCKW-Verbots-Verordnung in Kraft, die auch für den medizinischen Bereich die Verwendung limitiert.

Aus verschiedenen Forschungsarbeiten ist bekannt, daß nur die Chloratome in den FCKW und die daraus entstehenden freien Radikale für die Schädigung der Ozonschicht verantwortlich sind.

Es werden daher für technische, nicht pharmazeutische Anwendungen zunehmend sogenannte alternative Treibgase oder Treibmittel, z.B. gesättigte Kohlenwasserstoffe wie Propan, n-Butan, Isobutan oder Dimethylether, oder chlorfreie Fluorkohlenwasserstoffe (HFKW oder FKW) angewandt. Für pharmazeutisch-medizinische Anwendungen, d.h. als Treibgase für Aerosole zur Inhalation eignen sich jedoch nur solche Stoffe, die einerseits toxikologisch unbedenklich sind und möglichst keine Nebenwirkungen besitzen, andererseits sich aber gut mit den Wirkstoffen dieser Aerosole verarbeiten lassen und allen Anforderungen an die Qualität dieser Präparate genügen.

Charakterisiert werden die meisten Aerosole zur Inhalation in Druckgaspackungen dadurch, daß der Wirkstoff im verflüssigten Treibgas als suspendierte Partikel vorliegt. Bei Vitamin A handelt es sich jedoch um eine ölige, zäh-viskose Flüssigkeit, die sich im allgemeinen nicht suspendieren läßt. Die mit den FCKW-Treibmitteln bevorzugt angewandte Methode, den Wirkstoff im Treibgas zu lösen, scheitert jedoch an der Tatsache, daß aufgrund des niedrigen Dipolmomentes diese Treibmittel die Mischbarkeit der Treibgase mit dem Wirkstoff Vitamin A äußerst schlecht ist. Lösungsvermittler, wie Ethanol oder Ethylacetat, die bereits zur inhalativen Anwendung zugelassen sind, erhöhen zwar die Polarität der Treibgase, wirken sich jedoch in den lösungsvermittelnden Konzentrationen druckmindernd und somit negativ auf das für die Inhalation in tiefere Atemwege notwendige Partikelspektrum von ca. 0,5 - ca. 6 µm aus. Größere Tröpfchen und Teilchen gelangen nicht in die tieferen Atemwege aufgrund deren geometrischer Begrenzungen. Auch der Einsatz anderer, vor allem als Suspendierhilfsmittel für Inhalate verwendeter Stoffe, wie Sorbitantrioleat (SPAN 85), Lecithin oder Ölsäure, führt aufgrund der im konkreten Fall benötigten großen Einsatzmengen zur Partikelvergrößerung und damit nicht zum Erfolg.

Notwendig für den Ersatz von FCKW-Treibmitteln in Inhalations-Aerosolen mit dem Wirkstoff Vitamin A ist somit ein Stoff, der den zur Vernebelung des gelösten Wirkstoffes notwendigen Behälterinnendruck nicht entscheidend vermindert und mit dem Wirkstoff gut mischbar ist.

Überraschend zeigte sich, daß die gesättigten Kohlenwasserstoffe eben diese Eigenschaften besitzen.

Beispielsweise besitzt Isobutan bei 20°C einen Absolutdruck von 3,0 bar und somit einen genügend großen Aerosolisierungseffekt, um nahezu als alleiniges Treibgas zu dienen. Für medizinische Anwendungen, wo zur Inhalation in tiefere Atemwege ein sehr kleiner Tröpfchendurchmesser erforderlich ist, wird jedoch ein Zusatz eines Treibmittels mit noch höherem Druck benötigt. Dies kann beispielsweise HFKW 134a (Absolutdruck 5,7 bar) sein, jedoch hat sich überraschend gezeigt, daß auch HFKW 227 (Absolutdruck 4 bar) geeignet ist, in bestimmten Mischungsverhältnissen mit Isobutan einen genügend großen Aerosolierungseffekt zu erzeugen.

Neben Isobutan besitzen jedoch auch n-Butan, Propan und Dimethylether ähnliche Eigenschaften hinsichtlich des Lösungsverhaltens gegenüber Vitamin A sowie den Treibmitteln HFKW 134a und HFKW 227.

Der Verwendung von Isobutan, Propan und anderen gesättigten Kohlenwasserstoffen als Treibgase für Aerosole zur Inhalation standen bisher drei Faktoren entgegen.

Zum einen bemüht man sich im medizinischen Bereich den Einsatz brennbarer Treibgase soweit wie möglich zu vermeiden, da durch Fehlgebrauch, beispielsweise durch Sprühen des Inhalts in eine Flamme, Unfälle nicht auszuschließen sind. Zum anderen liegen zwar in erheblichen Umfang toxikologische Untersuchungen zu Kohlenwasserstofftreibmitteln wie Isobutan oder Propan vor, jedoch existiert zur Zeit in Deutschland oder der Europäischen Union kein Präparat zur Inhalation, das einen dieser Stoffe als Treibgas enthält. Deshalb wurde der Fachmann vom Einsatz abgehalten, da äußerst umfangreiches zusätzliches, toxikologisches Datenmaterial den Zulassungsbehörden vorzulegen ist. Demzufolge sind überwiegend nur technische Standardqualitäten dieser Rohstoffe im Handel, die aufgrund ihrer synthesebedingten Verunreinigungen und Begleitstoffe ein toxikologisches Risiko beinhalten.

Weiterhin besitzen diese Treibgase einen unvorteilhaften Eigengeschmack.

Werden jedoch Kohlenwasserstoff-Treibmittel nur als Lösungsvermittler eingesetzt, ist ihre Einsatzkonzentration in einer Druckgaspackung wesentlich niedriger. Dadurch ist sowohl die Brennbarkeit und somit auch die Unfallgefahr deutlich reduziert, als auch das potentielle toxikologische Risiko vermindert. Die Menge an Kohlenwasserstoff-Treibmittel muß nur so hoch gewählt werden, daß der Anteil an Wirkstoffen und gegebenenfalls an Hilfsstoffen klar gelöst vorliegt. Dadurch wird der für Kohlenwasserstoff-Treibmittel typische sclechte Eigengeschmack deutlich reduziert.

Gegenstand der Erfindung sind somit pharmazeutische Zusammensetzungen in Form eines Aerosols zur Anwendung auf Schleimhäuten der Atemwege, enthaltend:
a) einen Wirkstoff oder eine Wirkstoffkombination aus der Gruppe der Retinoide und Carotinoide
b) ein verflüssigtes Treibgas oder Treibgasgemisch aus der Gruppe der partiell bis vollständig fluorierten, jedoch chlorfreien Kohlenwasserstoffe
c) ein verflüssigtes Treibgas oder Treibgasgemisch als Lösungsvermittler mit Aerosolisierungseffekt aus der Gruppe der gesättigten Kohlenwasserstoffe
d) gegebenenfalls antioxidativ wirkende und/oder weitere übliche pharmazeutische Hilfsstoffe (z.B. Geschmackskorrigenzien), welche sich für Aerosolformulierungen eignen.

In einer bevorzugten Ausführungsform enthält das Aerosol das Treibgas Tetrafluorethan (HFKW 134a) und/oder Heptafluorpropan (HFKW 227). Weiterhin sind in dieser bevorzugten Ausführungsform Wirkstoffe oder Wirkstoffkombinationen aus der Gruppe folgender Retinoide enthalten:

Retinol, Retinsäure, Ester des Retinols oder der Retinsäure, Betacaroten oder andere Carotinoide. Zusätzlich kann ein lipophiles Antioxidans zu Stabilisierungszwecken enthalten sein. Als Lösungsvermittler mit Aerosolisierungseffekt eignen sich bevorzugt Stoffe aus der Gruppe n-Butan, Isobutan und Propan.

Der Begriff pharmazeutische Zusammensetzung definiert ein Stoffgemisch, das sich für verschiedene Anwendungen als Aerosol im Bereich der Atemwege, vorzugsweise für Inhalationen bei Mensch und Tier, eignet und zur Behandlung von diversen Krankheiten verwendbar ist.

Ein Aerosol ist ein Stoffgemisch, das aus einem gasförmigen Dispersionsmittel und flüssigen oder festen dispersen Bestandteilen besteht. Als gasförmiges Dispersionsmittel können komprimierte oder nicht komprimierte Gase, wie z.B. Luft dienen. Die komprimierten Gase fungieren als Treibmittel oder Treibgase und können durch Druck verflüssigt sein und in druckdichte Behältnisse (Druckgaspackungen) abgefüllt werden, wo sie unter Überdruck in flüssigen Zustand erhalten werden. Die Druckgaspackungen sind mit einem Ventil, meist einem Dosierventil, verschlossen. Bei Betätigung des Ventils entweicht aus dem unter Überdruck stehenden Behältnis das eigentliche Aerosol mit dem dispergierten Wirkstoffen.

Ebenfalls Gegenstand der Erfindung ist die Verwendung der erfindungsgemäßen pharmazeutischen Zusammensetzungen zur Behandlung von Erkrankungen und Zustände, für die aufgrund der Indikationsstellung der darin enthaltenen Wirkstoffe oder Wirkstoff-Kombinationen ein Bedürfnis besteht. Die Behandlung erfolgt vorzugsweise durch orale oder nasale Inhalation bei Mensch und Tier.

Die folgenden Beispiele illustrieren die Erfindung.

In einem Ansatz-Druckbehälter werden der Wirkstoff oder die Wirkstoffe sowie gegebenenfalls ein Antioxidans und andere Hilfsstoffe, soweit erforderlich, eingewogen und in dem als Lösungsvermittler fungierenden verflüssigten Gas oder Gasgemisch gelöst. Nach Rühren wird die unter Druck stehende Lösung mit dem eigentlichen Treibgas versetzt und mit der üblichen Druckfülltechnik in mit einem Dosierventil verschlossenen Behälter mit einem Volumen von ca. 20 ml abgefüllt.

Die auf diese Weise hergestellte Zusammensetzung besteht aus:

| | | |
|---|---|---|
| a) | 0,01 - 50 Gew.-% | Wirkstoff |
| b) | 40 - 99,49 Gew.-% | Treibgas |
| c) | 0,5 - 49,99 Gew.-% | Lösungsvermittler und |
| d) | 0 - 5 Gew.-% | Hilfsstoffe |

Vorzugsweise besteht sie aus:

| | | |
|---|---|---|
| a) | 0,01 - 30 Gew.-% | Wirkstoff |
| b) | 50 - 99 Gew.-% | Treibgas |
| c) | 1 - 49,99 Gew.-% | Lösungsvermittler und |
| d) | 0 - 3 Gew.-% | Hilfsstoffe |

Geeignete Zusammensetzungen sind:

| | | |
|---|---|---|
| a) | 0,05 - 20 Gew.-% | Wirkstoff |
| b) | 50 - 95 Gew.-% | Treibgas |
| c) | 5 - 45 Gew.-% | Lösungsvermittler und |
| d) | 0,01 - 2 Gew.-% | Hilfsstoffe; |
| a) | 0,01 - 10 Gew.-% | Wirkstoff |
| b) | 55 - 90 Gew.-% | Treibgas |
| c) | 10 - 45 Gew.-% | Lösungsvermittler und |
| d) | 0,1 - 2 Gew.-% | Hilfsstoffe |

Insbesondere vorteilhaft sind Zusammensetzungen aus:

| | | |
|---|---|---|
| a) | 0,1 - 8 Gew.-% | Wirkstoff |
| b) | 55 - 80 Gew.-% | Treibgas |
| c) | 15 - 45 Gew.-% | Lösungsvermittler und |
| d) | 0,01 - 1 Gew.-% | Hilfsstoffe |

In den folgenden, nicht beschränkenden Rezepturbeispielen ist die Einwaage jeweils in Gew.-% angegeben:

| Rezepturbelspiel | 1 | 2 | 3 | 4 | 5 | 6 | 7 | 8 |
|---|---|---|---|---|---|---|---|---|
| Retinolpalmitat | 1,10 | 3,19 | 5,42 | 7,35 | 3,22 | 0,72 | 2,46 | 2,84 |
| DL-α-Tocopherol | 0,11 | 0,27 | 0,54 | 0,74 | 0,32 | 0,07 | 0,25 | 0,24 |
| Tetrafluorethan | 76,71 | 69,22 | 57,87 | 55,15 | 64,31 | | | |
| Heptafluorpropan | | | | | | 71,89 | 73,89 | 57,34 |
| Isobutan | 22,08 | 27,32 | 36,17 | 36,76 | 32,15 | 27,32 | 23,40 | 39,58 |

Die Vorteile der vorliegenden Erfindung liegen somit darin, daß es ermöglicht wird, Lösungsaerosole, bei denen ein oder mehrere lipophile Wirkstoffe im verflüssigten Treibgas gelöst vorliegen sollen, herzustellen, obwohl die Wirkstoffe aus der Gruppe der Retinoide und Carotinoide nicht in den ozonunschädlichen, fluorierten Treibgasen löslich sind. Dieser Effekt wird durch die Verwendungvon niedriger Mengen gesättigter Kohlenwasserstoffe als Lösungsvermittler erzielt.

## Patentansprüche

1. Pharmazeutische Zusammensetzungen in Form eines Aerosols zur Anwendung auf Schleimhäuten der Atemwege, enthaltend:
a) einen Wirkstoff oder eine Wirkstoffkombination aus der Gruppe der Retinoide und Carotinoide,
b) ein verflüssigtes Treibgas oder Treibgasgemisch aus der Gruppe der partiell bis vollständig fluorierten, jedoch chlorfreien Kohlenwasserstoffe,
c) ein verflüssigtes Treibgas oder Treibgasgemisch als Lösungsvermittler mit Aerosolisierungseffekt aus der Gruppe der gesättigten Kohlenwasserstoffe,
d) gegebenenfalls antioxidativ wirkende und/oder weitere übliche pharmazeutische Hilfsstoffe, welche sich für Aerosolformulierungen eignen.

2. Pharmazeutische Zusammensetzungen gemäß Anspruch 1 enthaltend:
a) einen Wirkstoff oder eine Wirkstoffkombination aus der Gruppe Retinol, Retinsäure, Ester des Retinols oder der Retinsäure, Betacaroten,
b) ein verflüssigtes Treibgas oder Treibgasgemisch aus der Gruppe der partiell bis vollständig fluorierten, jedoch chlorfreien Kohlenwasserstoffe,
c) ein verflüssigtes Treibgas oder Treibgasgemisch als Lösungsvermittler mit Aerosolisierungseffekt aus der Gruppe der gesättigten Kohlenwasserstoffe,
d) gegebenenfalls antioxidativ wirkende und/oder weitere übliche pharmazeutische Hilfsstoffe, welche sich für Aerosolformulierungen eignen.

3. Pharmazeutische Zusammensetzungen gemäß Anspruch 1 enthaltend:
a) einen Wirkstoff oder eine Wirkstoffkombination aus der Gruppe der Retinoide und Carotinoide,
b) ein Treibgas oder Treibgasgemisch aus der Gruppe Tetrafluorethan (HFKW 134a) und Heptafluorpropan (HFKW 227),
c) ein verflüssigtes Treibgas oder Treibgasgemisch als Lösungsvermittler mit Aerosolisierungseffekt aus der Gruppe der gesättigten Kohlenwasserstoffe,
d) gegebenenfalls antioxidativ wirkende und/oder weitere übliche pharmazeutische Hilfsstoffe, welche sich für Aerosolformulierungen eignen.

4. Pharmazeutische Zusammensetzung gemäß Anspruch 1 enthaltend:
a) einen Wirkstoff oder eine Wirkstoffkombination aus der Gruppe der Retinoide und Carotinoide,
b) ein verflüssigtes Treibgas oder Treibgasgemisch aus der Gruppe der partiell bis vollständig fluorierten, jedoch chlorfreien Kohlenwasserstoffe,
c) ein Treibgas oder Treibgasgemisch als Lösungsvermittler mit Aerosolisierungseffekt aus der Gruppe n-Butan, Isobutan, Dimethylether und Propan,
d) gegebenenfalls antioxidativ wirkende und/oder weitere übliche pharmazeutische Hilfsstoffe, welche sich für Aerosolformulierungen eignen.

5. Pharmazeutische Zusammensetzung gemäß Anspruch 1 enthaltend:
a) einen Wirkstoff oder eine Wirkstoffkombination aus der Gruppe der Retinoide und Carotinoide,
b) ein verflüssigtes Treibgas oder Treibgasgemisch aus der Gruppe der partiell bis vollständig fluorierten, jedoch chlorfreien Kohlenwasserstoffe,
c) ein Treibgas oder Treibgasgemisch als Lösungsvermittler mit Aerosolisierungseffekt aus der Gruppe n-Butan, Isobutan und Propan,
d) gegebenenfalls antioxidativ wirkende und/oder weitere übliche pharmazeutische Hilfsstoffe, welche sich für Aerosolformulierungen eignen.

6. Pharmazeutische Zusammensetzung gemäß Anspruch 1 enthaltend:
a) einen Wirkstoff oder eine Wirkstoffkombination aus der Gruppe Retinol, Retinsäure, Ester des Retinols oder der Retinsäure und Betacaroten,
b) ein Treibgas oder Treibgasgemisch aus der Gruppe Tetrafluorethan (HFKW 134a) und Heptafluorpropan (HFKW 227),
c) ein Treibgas oder Treibgasgemisch als Lösungsvermittler mit Aerosolisierungseffekt aus der Gruppe n-Butan, Isobutan und Propan,
d) gegebenenfalls antioxidativ wirkende und/oder weitere übliche pharmazeutische Hilfsstoffe, welche sich für Aerosolformulierungen eignen.

7. Verwendung der Komponenten a-d gemäß einem der Ansprüche 1-6 zur Herstellung einer pharmazeutischen Zusammensetzung zur Vorbeugung und Behandlung von Schleimhauterkrankungen von Mensch und Tier.

8. Verwendung nach Anspruch 7 zur Vorbeugung und Behandlung von Funktionsanomalien, Erkrankungen und krankhaften Veränderungen an den Schleimhäuten des Tracheo-Bronchialtraktes von Mensch und Tier.

9. Verwendung nach Anspruch 7 oder 8 zur Vorbeugung und Behandlung von zellulären Differenzierungsstörungen der Schleimhäute des Tracheo-Bronchialtraktes, Plattenepithel-Metaplasien unabhängig von der Genese, neoplastischen Veränderungen, eingeschränkter Aktivität des Flimmerepithels und Dysfunktion schleimbildender Zellen unabhängig von der Genese.

10. Verwendung nach einem der vorhergehenden Ansprüche zur Therapie oder als topisches Adjuvans bei der Therapie von Bronchialcarcinomen, akuten und chronischen Bronchitiden, akuten und chronischen Funktionseinschränkungen infolge einer durch das Einatmen schleimhautschädigender Staube oder Gase aufgetretener Beeinträchtigung des Tracheobronchial-Epithels, und der bronchiopulmonalen Dysplasie von Neugeborenen.
